# EUROPÄISCHE PATENTSCHRIFT

(11) **EP 1 144 996 B1**
(45) Veröffentlichungstag und Bekanntmachung des Hinweises auf die Patenterteilung: **30.07.2003**
(21) Anmeldenummer: 99950366.7
(22) Anmeldetag: 30.04.1999
(51) Int. Cl.: G01N 33/18

(54) **AUTOMATISCHE BESTIMMUNG DER BELASTUNG VON WÄSSRIGEN REINIGUNGSLÖSUNGEN MIT KOHLENSTOFFHALTIGEN VERBINDUNGEN**
AUTOMATIC DETERMINATION OF THE CONTAMINATION OF AQUEOUS CLEANING SOLUTIONS WITH CARBONACEOUS COMPOUNDS
DETERMINATION AUTOMATIQUE DE LA SOLLICITATION DE SOLUTIONS NETTOYANTES AQUEUSES AVEC DES COMPOSES CONTENANT DU CARBONE

(30) Priorität: 09.05.1998 DE 19820800
(43) Veröffentlichungstag der Anmeldung: 17.10.2001
(73) Patentinhaber: Henkel Kommanditgesellschaft auf Aktien, 40589 Düsseldorf (DE)
(72) Erfinder: BARTIK-HIMMLER, Ibolya, D-51519 Odenthal (DE); WIMSCHNEIDER, Andrea, D-40597 Düsseldorf (DE); KREY, Wolfgang, D-42113 Wuppertal (DE); OPITZ, Werner, D-40764 Langenfeld (DE); KLING, Hans-Willi, D-42277 Wuppertal (DE); SCHENZLE, Bernd, D-40699 Erkrath (DE)
(86) Internationale Anmeldenummer: EP9902941
(87) Internationale Veröffentlichungsnummer: WO99058969

(56) Entgegenhaltungen:
- US-A- 3 854 881
- US-A- 4 207 450
- US-A- 5 312 756
- US-A- 5 677 190
- PATENT ABSTRACTS OF JAPAN vol. 1998, no. 11, 30. September 1998 (1998-09-30) & JP 10 177016 A (NIPPON PARKERIZING CO LTD), 30. Juni 1998 (1998-06-30)
- PATENT ABSTRACTS OF JAPAN vol. 1997, no. 07, 31. Juli 1997 (1997-07-31) & JP 09 072894 A (ARAKAWA CHEM IND CO LTD), 18. März 1997 (1997-03-18)
- PATENT ABSTRACTS OF JAPAN vol. 1997, no. 08, 29. August 1997 (1997-08-29) & JP 09 089638 A (HITACHI CABLE LTD), 4. April 1997 (1997-04-04)

## Beschreibung

Die Erfindung betrifft ein Verfahren zur automatischen Kontrolle und Steuerung von Reinigerbäder, wobei als Meß- und Steuerparameter der Gehalt der wäßrigen Reinigungslösung an anorganisch ("inorganic carbon", IC) oder organisch gebundenem Kohlenstoff ("total organic carbon", TOC) oder deren Summe ("total carbon", TC) bestimmt wird. Das Verfahren ist insbesondere für technische Reinigerbäder in der metallverarbeitenden Industrie wie beispielsweise im Automobilbau konzipiert. Es ermöglicht beispielsweise, die durch den Parameter TOC charakterisierte Belastung des Reinigerbades insbesondere mit Fetten und Ölen automatisch zu überwachen und erforderlichenfalls automatisch oder durch externe Anforderung das Reinigerbad zu ergänzen oder sonstige Badpflegemaßnahmen einzuleiten. Das Verfahren ist insbesondere so konzipiert, daß die Ergebnisse der Bestimmungen an einen vom Reinigerbad entfernten Ort übermittelt werden. Außerdem kann von einem vom Reinigerbad entfernten Ort aus in den automatischen Meßablauf eingegriffen werden oder die Nachdosierung oder sonstige Badpflegemaßnahmen angestoßen werden. Der "von dem Reinigerbad entfernten Ort" kann beispielsweise in einem übergeordneten Prozeßleitsystem, in einer Leitzentrale des Werkes, in dem sich das Reinigerbad befindet, oder auch an einer Stelle außerhalb des Werkes liegen.

Die Reinigung von Metallteilen vor ihrer Weiterverarbeitung stellt eine Standardaufgabe in der metallverarbeitenden Industrie dar. Die Metallteile können beispielsweise mit abgelösten oder ausgelaugten temporären Beschichtungen, Pigmentschmutz, Staub, Metallabrieb, Korrosionsschutzölen, Fügewerkstoffen wie beispielsweise Kleberresten, Kühlschmierstoffen oder Umformhilfsmitteln verschmutzt sein. Vor der Weiterverarbeitung wie insbesondere vor einer Korrosionsschutzbehandlung oder -beschichtung (z.B. Phosphatierung, Chromatierung, Anodisierung, Reaktion mit komplexen Fluoriden, organische Beschichtung usw.) oder vor einer Lackierung müssen diese Verunreinigungen durch eine geeignete Reinigungslösung entfernt werden. Hierfür kommen Spritz-, Tauch- oder kombinierte Verfahren in Frage.

Industrielle Reiniger in der metallverarbeitenden Industrie sind in der Regel alkalisch (pH-Werte im Bereich oberhalb 7, beispielsweise 9 bis 12), können jedoch auch sauer sein. Grundbestandteile alkalischer Reiniger sind Alkalien (Alkalimetallhydroxide, -carbonate, -silicate, -phosphate, -borate) sowie nichtionische und/oder anionische Tenside. Häufig enthalten die Reiniger als zusätzliche Hilfskomponenten Komplexbildner (Gluconate, Polyphosphate, Salze von Aminocarbonsäuren wie beispielsweise Ethylendiamintetraacetat oder Nitrilotriacetat, Salze von Phosphonsäuren wie beispielsweise Salze von Hydroxyethandiphosphonsäure, Phosphono-butantricarbonsäure, oder andere Phosphon- oder Phosphonocarbonsäuren), Korrosionsschutzmittel wie beispielsweise Salze von Carbonsäuren mit 6 bis 12 C-Atomen, Alkanolamine und Schauminhibitoren wie beispielsweise endgruppenverschlossene Alkoxylate von Alkoholen mit 6 bis 16 C-Atomen im Alkylrest. Sofern die Reinigerbäder keine anionischen Tenside enthalten, können auch kationische Tenside eingesetzt werden. Saure Reiniger enthalten anstelle der Alkalien Säuren wie beispielsweise Phosphorsäure oder Schwefelsäure.

Als nichtionische Tenside enthalten die Reiniger in der Regel Ethoxylate, Propoxylate und/oder Ethoxylate/Propoxylate von Alkoholen oder Alkylaminen mit 6 bis 16 C-Atomen im Alkylrest, die auch endgruppenverschlossen sein können. Als anionische Tenside sind Alkylsulfate und Alkylsulfonate weit verbreitet. Auch Alkylbenzolsulfonate sind noch anzutreffen, aus Umweltgesichtspunkten jedoch nachteilig. Als kationische Tenside kommen insbesondere kationische Alkylammoniumverbindungen mit mindestens einem Alkylrest mit 8 oder mehr C-Atomen in Frage.

Als Ergebnis des Reinigungsvorgangs reichern sich die von den Oberflächen abgelösten Schmutzbestandteile in der Reinigungslösung an. Pigmentschmutz kann zu einer Belastung mit anorganisch gebundenem Kohlenstoff führen. Korrosionsschutzöle, Kühlschmierstoffe oder Umformhilfsmittel wie beispielsweise Ziehfette und/oder abgelöste oder ausgelaugte organische Beschichtungen oder Fügewerkstoffe führen zu einer Belastung der Reinigungslösung mit organisch gebundenem Kohlenstoff. Da dieser organisch gebundene Kohlenstoff größtenteils in Form von Mineralölen, Mineralfetten oder von Ölen und Fetten tierischen oder pflanzlichen Ursprungs vorliegt, wird häufig kurz von der "Fettbelastung" der Reinigungslösung gesprochen. Solche Öle und Fette liegen in der Reinigungslösung größtenteils in emulgierter Form vor. Öle und Fette tierischen oder pflanzlichen Ursprungs können von einer alkalischen Reinigungslösung jedoch zumindest teilweise hydrolisiert werden. Die Hydrolyseprodukte können dann auch in gelöster Form in der Reinigungslösung vorliegen. Bei einer zu hohen TOC-Belastung der Reinigungslösung ist nicht mehr gewährleistet, daß sie die zu reinigenden Teile im erforderlichen Maße von Ölen und Fetten befreit. Oder es besteht die Gefahr, daß Öle und Fette auf die gereinigten Teile wieder aufziehen, wenn diese aus der Reinigungslösung entfernt werden. Daher ist es erforderlich, die Fettbelastung der Reinigungslösung unterhalb eines kritischen Höchstwerts zu halten, der von der Weiterverwendung der gereinigten Teile und von der Zusammensetzung der Reinigungslösung abhängen kann. Bei hoher Fettbelastung kann entweder der Tensidgehalt der Reinigungslösung erhöht werden, um das Fettlösevermögen der Reinigungslösung zu erhöhen. Oder man leitet Badpflegemaßnahmen mit dem Ziel ein, die Fettbelastung der Reinigungslösung zu verringern. Bei einer vorgegebenen Hochstgrenze der Fettbelastung ist dies auf jeden Fall erforderlich. Im einfachsten Falle verwirft man die Reinigungslösung ganz oder teilweise und setzt sie neu an oder ergänzt sie mit frischer Reinigungslösung. Wegen des hierbei anfallenden Abwassers sowie wegen des Bedarfs an Frischwasser ist man jedoch bemüht, Fette und Öle von der Reinigungslösung abzutrennen und die Reinigungslösung, ggf. nach Ergänzung mit Wirkstoffen, weiter einzusetzen. Geeignete Einrichtungen hierzu wie beispielsweise Separatoren oder Membranfiltrationsanlagen sind in der Technik bekannt.

Bisher war es üblich, die Reinigungsleistung einer Reinigungslösung visuell anhand des Reinigungsergebnisses zu beurteilen. Das Bedienungspersonal der Anlage beurteilt die Reinigungsleistung und ergreift die erforderlichen Maßnahmen wie beispielsweise Badergänzung oder Baderneuerung. Dieses derzeit übliche Verfahren setzt voraus, daß sich zu den erforderlichen Kontrollzeiten Bedienungspersonal in der Nähe des Reinigungsbades aufhält. Je kürzere Kontrollintervalle erwünscht werden, desto stärker wird das Bedienungspersonal für die visuelle Beurteilung beansprucht.

Die Erfindung stellt sich demgegenüber die Aufgabe, eine Kontrolle von Reinigerbäder durch Bestimmung des Gehalts an anorganisch und/oder organisch gebundenem Kohlenstoff derart automatisiert durchzuführen und zu dokumentieren, daß zumindest die Ergebnisse der Bestimmung auf einem Datenträger gespeichert und/oder ausgegeben werden. Vorzugsweise soll sich die eingesetzte Meßeinrichtung selbst überprüfen und kalibrieren und bei Fehlfunktion eine Alarmmeldung an einen entfernten Ort übermitteln. Weiterhin soll es vorzugsweise möglich sein, die Funktionsfähigkeit der Meßeinrichtung und die Meßergebnisse von einem entfernten Ort aus zu überprüfen. Weiterhin soll von einem entfernten Ort aus in den Meßablauf und in die Badpflegemaßnahmen eingegriffen werden können. Durch die angestrebte Fernkontrolle soll der personelle Aufwand für die Badkontrolle und die Badsteuerung der Reinigerbäder verringert werden.

Diese Aufgabe wird gelöst durch ein Verfahren, wie im Anspruch 1 definiert.

Das im Teilschritt a) gezogene Probenvolumen kann dem Steuerteil der für das Verfahren einzusetzenden Meßeinrichtung fest einprogrammiert sein. Vorzugsweise ist die Größe des Probenvolumens von einem entfernten Ort aus änderbar. Weiterhin kann das Steuerprogramm so ausgelegt sein, daß es das zu verwendende Probevolumen von dem Ergebnis einer vorausgehenden Messung abhängig macht und/oder die Probe automatisch auf einen günstigen Meßbereich verdünnt. Beispielsweise kann das Probenvolumen um so größer gewählt werden, je geringer die Fettbelastung des Reinigerbades ist. Die Genauigkeit der Bestimmung kann hierdurch optimiert werden.

Wenn im Sinne des erfindungsgemäßen Verfahrens von einem "entfernten Ort" die Rede ist, so ist damit ein Ort gemeint, der sich nicht im unmittelbaren oder zumindest im optischen Kontakt mit dem Reinigerbad befindet. Der entfernte Ort kann beispielsweise ein zentrales Prozeßleitsystem darstellen, das im Rahmen eines Gesamtverfahrens zur Oberflächenbehandlung der Metallteile als Teilaufgabe das Reinigerbad kontrolliert und steuert. Der "entfernte Ort" kann auch eine zentrale Leitwarte darstellen, von der aus der Gesamtprozeß kontrolliert und gesteuert wird und die sich beispielsweise in einem anderen Raum als das Reinigerbad befindet.

Als "entfernter Ort" kommt jedoch auch eine Stelle außerhalb des Werkes in Betracht, in dem sich das Reinigerbad befindet. Hierdurch wird es möglich, daß Spezialisten das Reinigerbad überprüfen und steuern, die sich außerhalb des Werkes aufhalten, in dem sich das Reinigerbad befindet. Hierdurch ist es wesentlich seltener erforderlich, daß sich Spezialpersonal am Ort des Reinigerbades aufhält.

Geeignete Datenleitungen, mit denen sich die Ergebnisse der Bestimmungen sowie Steuerbefehle übertragen lassen, stehen im Stand der Technik zur Verfügung.

Zwischen dem Ziehen der Probe und der eigentlichen Messung kann es wünschenswert sein, die Probe im fakultativen Teilschritt b) von Feststoffen zu befreien. Bei einem nur wenig mit Feststoffen belasteten Reinigerbad ist dieses nicht erforderlich. Bei einem zu hohen Feststoffgehalt des Reinigerbades können jedoch Ventile der Meßeinrichtung verstopfen. Daher ist es empfehlenswert, Feststoffe aus der Probe zu entfernen. Dies kann automatisch durch Filtration oder auch durch Verwendung eines Zyklons oder einer Zentrifuge erfolgen. Empfehlenswert ist es, die Probe beispielsweise durch kräftiges Rühren zu homogenisieren. Hierdurch wird eine gleichmäßige und feine Verteilung der ggf. als grobe Öl- oder Fettröpfchen vorliegenden organischen Verunreinigungen erreicht.

Falls erforderlich, wird im Teilschritt c) die Probe in einem bestimmten Verhältnis mit Wasser verdünnt. Dieses Verhältnis kann fest vorgegeben, jedoch von einem entfernten Ort aus änderbar sein. Das Verdünnungsverhältnis kann jedoch auch von dem Ergebnis einer vorhergegangenen Bestimmung des Gehalts an anorganisch und/oder organisch gebundenem Kohlenstoff abhängig gemacht werden. Hierdurch wird gewährleistet, daß der Kohlenstoffgehalt der Probelösung in einem Bereich liegt, der eine optimale Bestimmung mit der gewählten Methode erlaubt.

Im Teilschritt d) kann der anorganisch und/oder organisch gebundene Kohlenstoff beispielsweise dadurch bestimmt werden, daß man ihn in CO₂ überführt und das gebildete CO₂ quantitativ bestimmt.

Die Überführung des Kohlenstoffs zu CO₂ durch Oxidation kann beispielsweise durch Verbrennung bei erhöhter Temperatur in der Gasphase erfolgen. Die erhöhte Temperatur bei der Verbrennung liegt vorzugsweise oberhalb von etwa 600 °C, beispielsweise bei etwa 680 °C. Vorzugsweise erfolgt die Verbrennung mit Luft oder mit Sauerstoffgas in einem Reaktionsrohr mit Hilfe eines Katalysators. Als Katalysator kommen beispielsweise Edelmetalloxide oder andere Metalloxide wie beispielsweise Vanadate, Vanadiumoxide, Chrom-, Mangan- oder Eisenoxide in Betracht. Auch auf Aluminiumoxid niedergeschlagenes Platin oder Palladium kann als Katalysator eingesetzt werden. Nach diesem Verfahren erhält man direkt ein CO₂-haltiges Verbrennungsgas, dessen CO₂-Gehalt wie nachstehend beschrieben bestimmt werden kann.

Alternativ zu einer Verbrennung in der Gasphase kann die Überführung des Kohlenstoffs in CO₂ auch naßchemisch erfolgen. Hierbei oxidiert man den Kohlenstoff der Probe mit einem starken chemischen Oxidationsmittel wie beispielsweise mit Wasserstoffperoxid oder mit Peroxodisulfat. Diese naßchemische Oxidationsreaktion kann erwünschtenfalls mit Hilfe eines Katalysators der vorstehend genannten Art und/oder mit UV-Strahlung beschleunigt werden. In diesem Falle ist es vorzuziehen, das entstandene CO₂ mit einem Gasstrom aus der - erforderlichenfalls angesäuerten - Probe auszutreiben, um es quantitativ zu bestimmen. Dabei kann in Form von Carbonaten oder von CO₂ gebundener Kohlenstoff ebenfalls erfaßt werden.

Unabhängig von der Methode, mit der gasförmiges CO₂ erzeugt wurde, kann dieses nach einer der folgenden Methoden quantitativ bestimmt werden. Bei bekannter Probenmenge kann hieraus der Gehalt der Reinigungslösung an anorganisch und/oder organisch gebundenem Kohlenstoff errechnet werden. Alternativ wird durch einen vorgegebenen Umrechnungsfaktor das Ergebnis der Bestimmung als Fettbelastung pro 1 Reinigerbad angegeben, wenn anorganisch gebundener Kohlenstoff nicht vorhanden ist oder vorher entfernt wurde.

Für die Bestimmung des CO₂-Gehalts des erhaltenen Gasstroms kommen unterschiedliche, im Stand der Technik bekannte Methoden in Frage. Beispielsweise kann man die Gase durch eine Absorberlösung leiten und beispielsweise die Gewichtszunahme der Absorberlösung messen. Geeignet ist hierfür beispielsweise eine wäßrige Lösung von Kaliumhydroxid, die CO₂ unter Bildung von Kaliumcarbonat aufnimmt. Alternativ zur Bestimmung der Gewichtszunahme kann die Änderung der elektrischen Leitfähigkeit der Absorptionslösung oder ihre Restalkalität nach Absorption des CO₂ bestimmt werden.

Das gebildete CO₂ kann auch an einem geeigneten Festkörper absorbiert werden, dessen Gewichtszunahme man mißt. Geeignet hierfür ist beispielsweise Natronasbest. Selbstverständlich müssen sowohl eine Absorberlösung als auch ein Feststoffabsorber ausgetauscht werden, wenn sie erschöpft sind und kein CO₂ mehr binden können.

Für ein automatisch arbeitendes Verfahren ist es jedoch einfacher, den CO₂-Gehalt des Gases durch Messung der Infrarotabsorption quantitativ zu bestimmen. Die Bestimmung der Infrarotabsorption kann beispielsweise bei einer Wellenlänge von 4,26 µm entsprechend einer Wellenzahl von 2349 cm⁻¹ durchgeführt werden. Geräte, die die Verbrennung der Probe und die Messung der Infrarotabsorption durchführen können, sind im Stand der Technik bekannt. Beispielsweise genannt sei das TOC-System der Firma Shimadzu.

Für die photometrische Bestimmung des CO₂-Gehalts des Verbrennungsgases bzw. des aus der Probe ausgetriebenen Gases kommen nicht nur dispersiv arbeitende Infrarotspektrometer, sondern auch nichtdispersive Photometer in Betracht. Diese sind auch als "NDIR-Geräte" bekannt. Ein derartiges Gerät ist beispielsweise in der DE-A-44 05 881 beschrieben.

Bei dieser Bestimmungsmethode wird auch derjenige Anteil an Kohlenstoff erfaßt, der auf bewußt zugesetzte Wirkstoffe in der Reinigungslösung zurückgeht. Beispielsweise seien hier Tenside, organische Korrosionsinhibitoren und organische Komplexbildner genannt. Deren Gehalt in der Reinigungslösung ist jedoch innerhalb gewisser Schwankungsgrenzen bekannt oder kann getrennt bestimmt werden. Der auf diese Wirkstoffe zurückgehende Anteil an organisch gebundenem Kohlenstoff kann also vom Ergebnis der Bestimmung abgezogen werden. Man erhält dann den Anteil, der auf die eingetragenen Verunreinigungen zurückzuführen ist. Dabei ist es in der Praxis nicht unbedingt erforderlich, den in Form von Wirkstoffen vorliegenden Kohlenstoffanteil bei der Kohlenstoff-Bestimmung zu berücksichtigen. Vielmehr genügt es häufig, eine Obergrenze des Kohlenstoff-Gehalts der Reinigungslösung festzulegen, die den Wirkstoffgehalt bereits berücksichtigt. Durch die Kohlenstoff-Bestimmung wird dann festgestellt, ob die Kohlenstoff-Belastung unterhalb oder oberhalb dieser Höchstgrenze liegt.

Der in Form lipophiler Stoffe vorliegenden Anteil an organisch gebundenem Kohlenstoff kann alternativ so bestimmt werden, daß man die lipophilen Stoffe in ein mit Wasser nicht in jedem Verhältnis mischbares organisches Lösungsmittel extrahiert. Nach Abdampfen des Lösungsmittels bleiben die lipophilen Stoffe zurück und können gravimetrisch bestimmt werden. Vorzugsweise geht man jedoch so vor, daß man die Infrarotabsorption der lipophilen Stoffe im Extrakt photometrisch bestimmt. Hierbei kommen als mit Wasser nicht in jedem Verhältnis mischbares organisches Lösungsmittel insbesondere halogenierte Kohlenwasserstoffe in Betracht. Ein bevorzugtes Beispiel hierfür ist 1,1,2-Trichlortrifluorethan. Diese Bestimmungsmethode ist angelehnt an die DIN 38409, Teil 17. Im Gegensatz zu dieser Methode wird der Anteil lipophiler Stoffe in der Probe jedoch nicht gravimetrisch nach Abdampfen des organischen Lösungsmittels, sondern photometrisch im organischen Lösungsmittel bestimmt. Die quantitative Bestimmung erfolgt vorzugsweise wie in der DIN 38409, Teil 18 durch Messung der Infrarotabsorption der lipophilen Stoffe im Extrakt bei einer charakteristischen Schwingungsfrequenz der CH₂-Gruppe. Dabei ist es empfehlenswert, ein solches organisches Lösungsmittel zur Extraktion zu verwenden, das selbst keine CH₂-Gruppen enthält. Beispielsweise kann für diese photometrische Bestimmung die Infrarot-Absorptionsbande bei 3,42 µm (2924 cm⁻¹) herangezogen werden. Hierbei werden alle organischen Substanzen erfaßt, die CH₂-Gruppen aufweisen und die in das organische Lösungsmittel extrahiert werden können. Teilweise sind dies auch die Tenside in der Reinigungslösung. Sofern deren Anteil nicht mit erfaßt werden soll, ist er getrennt durch eine Alternativmethode zu bestimmen und vom Gesamtergebnis abzuziehen. Erforderlichenfalls muß zuvor der Verteilungsquoiffizent der Tenside zwischen der Reinigungslösung und dem mit Wasser nicht in jedem Verhältnis mischbaren organischen Lösungsmittel bestimmt worden sein. In der Praxis kann es jedoch ausreichend sein, einen Höchstwert der zulässigen Belastung der Reinigungslösung mit lipophilen Stoffen festzulegen, der den Tensidanteil mit berücksichtigt. Wird dieser Höchstwert überschritten, sind Badpflegemaßnahmen einzuleiten.

Im Rahmen dieser Methode ist es empfehlenswert, daß Infrarotspektrometer mit einer bekannten Menge einer lipophilen Substanz zu kalibrieren. Als Kalibrierlösung eignet sich beispielsweise eine Lösung von 400 bis 500 mg Methylpalmitat in 100 ml 1,1,2-Trichlortrifluorethan. Diese Kalibrierlösung dient ebenfalls zur Funktionskontrolle des IR-Photometers.

Dabei geht man vorzugsweise so vor, daß man die Probe der Reinigungslösung zunächst mit einer phosphorsauren Magnesiumsulfatlösung versetzt. Diese Lösung bereitet man dadurch zu, daß man 220 g kristallines Magnesiumsulfat und 125 ml 85 gew.-%ige Phosphorsäure in deionisiertem Wasser löst und diese Lösung mit deionisiertem Wasser auf 1000 g auffüllt. Die Probelösung wird mit etwa 20 ml der phosphorsauren Magnesiumsulfatlösung versetzt. Anschließend gibt man 50 ml des mit Wasser nicht in jedem Verhältnis mischbaren organischen Lösungsmittels, vorzugsweise 1,1,2-Trichlortrifluorethan zu. Man vermischt die wäßrige und die organische Phase, führt eine Phasentrennung herbei und isoliert die organische Phase. Vorzugsweise wäscht man diese organische Phase nochmal mit der phosphorsauren Magnesiumsulfatlösung, führt erneut die Phasentrennung herbei und zieht die organische Phase ab. Diese wird in eine Meßküvette überführt und die Infrarotabsorption bei einer Schwingungsbande der CH₂-Gruppe vermessen. Als Meßküvette eignet sich beispielsweise eine Quarzglasküvette mit einer Schichtdicke von 1 mm. Aus dem Vergleich mit der Eichkurve, die auch den Blindwert des Photometeres enthält, kann der Gehalt an lipophilen Stoffen in der Probe anhand der Infrarotabsorption bestimmt werden.

Unabhängig von der Art der gewählten Bestimmungsmethode wird das Ergebnis der Bestimmung anschließend ausgegeben und/oder auf einem Datenträger gespeichert (Teilschritt e)). Dabei kann sich der Datenträger am Ort der Bestimmung oder auch in einer entfernten Recheneinheit befinden. Unter "Ausgabe des Ergebnisses der Bestimmung" wird verstanden, daß dieses entweder an ein übergeordnetes Prozeßleitsystem weitergegeben oder für einen Menschen erkennbar auf einem Bildschirm angezeigt oder ausgedruckt wird. Dabei kann der Ort der Anzeige bzw. Ausgabe des Ergebnisses der weiter oben definierte "entfernte Ort" sein. Es ist vorzuziehen, daß die Ergebnisse der einzelnen Bestimmungen zumindest für einen vorgegebenen Zeitintervall auf einem Datenträger gespeichert werden, so daß sie anschließend, beispielsweise im Sinne einer Qualitätssicherung, ausgewertet werden können. Die Ergebnisse der Kohlenstoff-Bestimmungen müssen jedoch nicht unmittelbar als solche ausgegeben oder auf Datenträger gespeichert werden. Vielmehr können sie auch direkt als Basis für weitere Berechnungen herangezogen werden, wobei die Ergebnisse dieser weiteren Berechnungen angezeigt oder gespeichert werden. Beispielsweise kann anstelle des jeweils aktuellen Kohlenstoff-Gehalts der Trend der Werte und/oder deren relative Änderung angezeigt werden. Oder die aktuellen Kohlenstoff-Gehalte werden in "% des Maximalgehalts" umgewandelt.

Im einfachsten Falle arbeitet das erfindungsgemäße Verfahren so, daß die Teilschritte a) bis e) nach einem vorgegebenen Zeitintervall wiederholt werden. Das vorgegebene Zeitintervall richtet sich dabei nach den Anforderungen des Betreibers des Reinigungsbades und kann jedes beliebige Zeitintervall im Bereich von wenigen Minuten bis zu mehreren Tagen umfassen. Für eine Qualitätssicherung ist es vorzuziehen, daß die vorgegebenen Zeitintervalle beispielsweise im Bereich zwischen 5 Minuten und 2 Stunden liegen. Beispielsweise kann man alle 15 Minuten eine Messung durchführen.

Das erfindungsgemäße Verfahren kann jedoch auch so durchgeführt werden, daß man die Schritte a) bis e) nach um so kürzeren Zeitintervallen wiederholt, je stärker sich die Ergebnisse zweier aufeinander folgender Bestimmungen unterscheiden. Das Steuersystem für das erfindungsgemäße Verfahren kann also selbst entscheiden, ob die Zeitintervalle zwischen den einzelnen Bestimmungen verkürzt oder verlängert werden sollen. Selbstverständlich muß dem Steuersystem die Anweisung vorgegeben werden, bei welchen Differenzen zwischen aufeinander folgenden Bestimmungen welche Zeitintervalle gewählt werden sollen. Es kann auch vorgesehen werden, daß die Meßintervalle an die Ergebnisse der Messung anderer Inhaltsstoffe gekoppelt werden. Beispielsweise können die Zeitintervalle, in denen der anorganisch und/oder organisch gebundene Kohlenstoff in der Reinigungslösung gemessen wird, von den Ergebnissen einer Messung des Tensidgehalts abhängig gemacht werden. Selbstverständlich können auch variable Meßintervalle extern vorgegeben werden, die beispielsweise mit dem Materialdurchsatz durch das Reinigungsbad und/oder mit der bekannten mittleren Verschmutzung des Reinigungsgutes korreliert sind.

Weiterhin kann das erfindungsgemäße Verfahren so durchgeführt werden, daß die Teilschritte a) bis e) zu jedem beliebigen Zeitpunkt aufgrund einer externen Anforderung ausgeführt werden. Hierdurch kann beispielsweise eine sofortige Kontrolle des Kohlenstoff-Gehalts des Reinigerbads vorgenommen werden, wenn in nachfolgenden Prozeßschritten Qualitätsprobleme festgestellt werden. Die Kohlenstoff-Messung kann also zeitgesteuert (nach festen Zeitintervallen) oder ereignisgesteuert (bei festgestellten Änderungen oder durch äußere Anforderungen) erfolgen.

Dabei legt man das Probennahme- und Meßsystem vorzugsweise so aus, daß einer zentralen Meßeinheit Proben aus unterschiedlichen Reinigungsbädern zugeführt werden können. Auf dem betroffenen Industriesektor ist es üblich, Metallteile in mehreren hintereinander geschalteten Reinigungsbädern zu reinigen. Durch Probenleitungen zu den einzelnen Reinigungsbädern können die Kohlenstoff-Gehalte der jeweiligen Reinigungslösungen mit einer einzigen Meßeinheit nacheinander bestimmt werden. Die Meßreihenfolge der einzelnen Bäder kann dabei extern vorgegeben werden. Dabei können unterschiedliche Meßintervalle für die einzelnen Reinigungsbäder vorgesehen werden, so daß beispielsweise ein bestimmtes Reinigungsbad öfter überprüft wird als ein anderes. Außerdem kann vorgesehen werden, daß der Kohlenstoff-Gehalt in einem nachgeschalteten Reinigerbad erst dann überprüft wird, wenn der Kohlenstoff-Gehalt in einem vorgeschalteten Reinigerbad einen bestimmten Grenzwert erreicht.

Bei der Durchführung des erfindungsgemäßen Verfahrens kann es erwünscht sein, sowohl anorganisch gebundenen Kohlenstoff als auch organisch gebundenen Kohlenstoff (TOC) zu erfassen. Dies ist beispielsweise der Fall, wenn zur Bestimmung des Kohlenstoffgehalts die Probe verbrannt wird. Hierbei wird auch gelöstes CO₂ oder in Form von Carbonaten vorliegender Kohlenstoff erfaßt, falls die Carbonate bei der gewählten Verbrennungstemperatur CO₂ abspalten. Soll in diesem Fall der anorganisch gebundene Kohlenstoff nicht mit erfaßt werden, kann er dadurch entfernt werden, daß man die Probe ansäuert und das gebildete CO₂ mit einem Gas wie beispielsweise Luft oder Stickstoff ausbläst. Dies kann erwünscht sein, wenn man speziell nur die "Fettbelastung" des Reinigerbads bestimmen will. Bei der Bestimmung des in Form lipophiler Stoffe vorliegenden Kohlenstoff-Gehalts nach der vorstehend beschriebenen Extraktionsmethode wird anorganisch gebundener Kohlenstoff automatisch nicht mit erfaßt.

Ebenso ist es möglich, flüchtige organische Verbindungen vor der Ausführung des Teilschritts d) durch Ausblasen mit einem Gas wie beispielsweise Luft oder Stickstoff aus der Probe zu entfernen. Beispielsweise können hierdurch flüchtige Lösungsmittel vor der Kohlenstoff-Bestimmung entfernt werden.

Vorzugsweise wird das erfindungsgemäße Verfahren so durchgeführt, daß sich die verwendete Meßeinrichtung selbst kontrolliert und erforderlichenfalls nachkalibriert. Hierzu kann vorgesehen werden, daß man nach einem vorgegebenen Zeitintervall oder nach einer vorgegebenen Anzahl von Bestimmungen oder aufgrund einer externen Anforderung durch Kontrollmessungen einer oder mehrerer Standardlösungen die Funktionsfähigkeit der verwendeten Meßeinrichtung überprüft. Zur Überprüfung wird eine Standardlösung mit bekannten Gehalten an anorganisch und/oder organisch gebundenem Kohlenstoff vermessen. Diese Überprüfung ist am realitätsnächsten, wenn man als Standardlösung eine Standardreinigungslösung einsetzt, deren Zusammensetzung der zu überprüfenden Reinigungslösung möglichst nahekommt. Standard-Lösungen, die keine Reinigungslösungen darstellen, können jedoch ebenfalls zur Überprüfung und/oder Kalibrierung herangezogen werden.

Ermittelt die Meßeinrichtung bei einer Kontrollmessung einer Standardlösung einen Kohlenstoff-Gehalt, der um einen vorzugebenden Mindestbetrag vom Sollgehalt abweicht, gibt die Meßeinrichtung lokal oder vorzugsweise an einem entfernten Ort eine Alarmmeldung aus. Dabei kann die Alarmmeldung durch einen vom Steuerprogramm der Meßeinrichtung oder dem übergeordneten Prozeßleitsystem ausgewählten Vorschlag zum Eingreifen enthalten.

Im erfindungsgemäßen Verfahren kann auch vorgesehen werden, daß man durch Kontrollmessung einer oder mehrerer Standardlösungen die Funktionsfähigkeit der verwendeten Meßeinrichtung überprüft, wenn die Ergebnisse zweier aufeinander folgender Messungen um einen vorgegebenen Betrag differieren. Hierdurch kann unterschieden werden, ob festgestellte Abweichungen im Kohlenstoff-Gehalt der Reinigungslösung real sind und Badpflegemaßnahmen erfordern oder ob sie durch einen Fehler im Meßsystem vorgetäuscht werden.

Je nach Ergebnis der Überprüfung der verwendeten Meßeinrichtung kann man die zwischen der aktuellen und der vorhergehenden Kontrollmessung erfolgten Bestimmungen des Gehalts an anorganisch und/oder organisch gebundenem Kohlenstoff mit einem Statuskennzeichen versehen, das die Zuverlässigkeit dieser Bestimmungen kennzeichnet. Haben beispielsweise aufeinander folgende Kontrollmessungen zur Überprüfung der verwendeten Meßeinrichtung ergeben, daß diese korrekt arbeitet, können die Bestimmungen des Kohlenstoff-Gehalts mit einem Statuskennzeichen "in Ordnung" versehen werden. Differieren die Ergebnisse der Kontrollmessungen um einen vorgegebenen Mindestbetrag, können beispielsweise die zwischenzeitlich erfolgten Bestimmungen mit dem Statuskennzeichen "zweifelhaft" versehen werden.

Weiterhin kann vorgesehen werden, daß man je nach Ergebnis der Überprüfung der verwendeten Meßeinrichtung mit der automatischen Bestimmung des Gehalts an anorganisch und/oder organisch gebundenem Kohlenstoff fortfährt und/oder eine oder mehrere der folgenden Aktionen durchführt: Analyse festgestellter Abweichungen, Korrektur der Meßeinrichtung, Beenden der Bestimmung des Kohlenstoff-Gehalts, Senden einer Statusmeldung oder eines Alarmsignals an ein übergeordnetes Prozeßleitsystem oder eine Überwachungseinrichtung, also an einen entfernten Ort. Die Meßeinrichtung kann also, falls erwünscht, nach vorgegebenen Kriterien selbst entscheiden, ob sie soweit funktionsfähig ist, daß mit den Kohlenstoff-Bestimmungen fortgefahren werden kann, oder ob Abweichungen festgestellt werden, die ein manuelles Eingreifen erforderlich machen.

Vorzugsweise ist das im erfindungsgemäßen Verfahren eingesetzte Meßsystem so ausgelegt, daß es die Füllstände und/oder den Verbrauch der verwendeten Standardund Testlösungen sowie evtl. Hilfslösungen automatisch überwacht und bei Unterschreiten eines vorgegebenen Mindestfüllstandes eine Warnmeldung ausgibt. Hierdurch kann vermieden werden, daß die Meßeinrichtung dadurch funktionsunfähig wird, daß ihr die erforderlichen Lösungen fehlen. Die Überwachung der Füllstände kann mit bekannten Methoden erfolgen. Beispielsweise können die Gefäße mit den Lösungen auf einer Waage stehen, die das jeweilige Gewicht der Lösungen registriert. Oder man setzt einen Schwimmer ein. Alternativ kann ein Mindestfüllstand durch eine Leitfähigkeitselektrode überprüft werden, die in das Gefäß mit der Lösung eintaucht. Die von der Meßeinrichtung auszugebende Warnmeldung wird vorzugsweise an den entfernten Ort übertragen, so daß von dort aus die entsprechenden Maßnahmen eingeleitet werden können. Generell ist in dem erfindungsgemäßen Verfahren vorzugsweise vorgesehen, daß man die Ergebnisse der Bestimmungen und/oder der Kontrollmessungen und/oder der Kalibrierungen und/oder die Statussignale kontinuierlich oder in vorgegebenen Zeitabständen und/oder auf Anforderung an einen entfernten Ort überträgt. Hierdurch ist Kontrollpersonal, das sich nicht am Ort des Reinigerbades befinden muß, laufend über dessen aktuellen Gehalt an anorganisch und/oder organisch gebundem Kohlenstoff und somit über die aktuelle Fett- und Ölbelastung informiert. Je nach Ergebnis der Bestimmungen und der Kontrollmessungen können entweder automatisch über ein Prozeßleitsystem oder durch manuelles Eingreifen erforderliche Korrekturmaßnahmen getroffen werden.

Die einfachste Korrekturmaßnahme besteht darin, daß man bei Überschreiten eines vorgegebenen Höchstwertes an anorganisch und/oder organisch gebundem Kohlenstoff oder auf externe Anforderung eine Einrichtung aktiviert, die eine oder mehrere Ergänzungskomponenten (Lösung oder Pulver) in das Reinigungsbad dosiert. Dies kann beispielsweise derart automatisiert erfolgen, daß je nach ermitteltem Kohlenstoff-Gehalt eine bestimmte Menge Ergänzungslösung oder Ergänzungspulver dem Reinigungsbad zugeführt wird. Hierbei können die Größe der Zugabeportion selbst oder bei fest vorgegebenen Zugabeportionen die Zeitintervalle zwischen den einzelnen Zugaben variiert werden. Dies kann beispielsweise über Dosierpumpen oder auch gewichtsgesteuert erfolgen. Im erfindungsgemäßen Verfahren ist also zum einen vorgesehen, daß bei bestimmten Abweichungen vom Sollwert (insbesondere, wenn durch die Kontrollmessungen die Funktionsfähigkeit der Meßeinrichtung feststeht) eine bestimmte Menge Ergänzungskomponente in das Reinigungsbad nachdosiert wird. Daneben kann vorgesehen werden, daß diese Maßnahmen zur Badergänzung dann durchgeführt werden, wenn eine vorgegebene Mindeständerung des Kohlenstoff-Gehalts festgestellt worden ist. Weiterhin kann dieses Nachdosieren jedoch auch aufgrund einer externen Anforderung, beispielsweise von einem entfernten Ort aus, unabhängig von dem aktuellen Kohlenstoff-Gehalt vorgenommen werden. Durch das Nachdosieren beispielsweise von Tensiden erhöht sich der Kohlenstoff-Gehalt der Reinigungslösung. Bei der nächsten Bestimmung des Kohlenstoff-Gehalts muß dies entsprechend berücksichtigt werden, was automatisch erfolgen kann. Durch eine Tensidzugabe erhöht sich das Öl- und Fettragevermögen des Reinigerbads. Entsprechend muß der tolerierbare Maximalwert der Kohlenstoff-Belastung angehoben werden, bei dessen Überschreiten die nächste Badpflegemaßnahme eingeleitet wird. Dies kann in dem Steuerprogramm automatisch vorgesehen werden.

Anstelle einer Nachdosierung von Badkomponenten wie beispielsweise Tensiden oder beim Überschreiten eines vorgegebenen Höchstgehalts an anorganisch und/oder organisch gebundenem Kohlenstoff können Badpflegemaßnahmen eingeleitet werden, die den Gehalt der Reinigungslösung an anorganisch und/oder organischen gebundenem Kohlenstoff verringern. Derartige Badpflegemaßnahmen haben insbesondere das Ziel, den Fett- und Ölgehalt der Reinigungslösung zu verringern. Im einfachsten Falle kann dies dadurch erfolgen, daß die Reinigungslösung ganz oder teilweise abgelassen und durch frische Reinigungslösung ersetzt wird. Ökonomischer ist es jedoch, Öle und Fette aus der Reinigungslösung durch im Stand der Technik bekannte Maßnahmen wie Abscheiden durch einen Separator oder Abtrennen durch Membranfiltration zu entfernen. Da bei diesen Verfahren auch Tenside zumindest teilweise ausgetragen werden, muß die Reinigungslösung entsprechend ergänzt werden. Auch das Einleiten dieser Maßnahmen kann nicht nur vom absoluten Kohlenstoff-Gehalt der Reinigerlösung abhängig gemacht werden, sondern von einer vorgegebenen Änderung des Kohlenstoff-Gehalts.

Selbstverständlich setzt das erfindungsgemäße Verfahren voraus, daß man die entsprechende Einrichtung zur Verfügung stellt. Diese enthält eine Steuerung, beispielsweise eine Rechnersteuerung, die zeit- und/oder ereignisabhängig den Meßverlauf steuert. Sie muß weiterhin die erforderlichen Gefäße für Lösungen, Rohrleitungen, Ventile, Dosier- und Meßeinrichtungen etc. zur Steuerung und Messung der Probenströme enthalten. Die Materialien sollen dem Verwendungszweck angepaßt sein, beispielsweise aus Edelstahl und/oder aus Kunststoff bestehen. Die Steuerelektronik der Meßeinrichtung sollte eine entsprechende input-output-Schnittstelle aufweisen, um mit einem entfernten Ort kommunizieren zu können.

Das erfindungsgemäße Verfahren ermöglicht es, zum einen den Kohlenstoff-Gehalt von Reinigungsbädern vor Ort zu überprüfen und ohne manuellen Eingriff vorgegebene Korrekturmaßnahmen einzuleiten. Hierdurch wird die Prozeßsicherheit erhöht und ein konstant zuverlässiges Reinigungsergebnis erzielt. Abweichungen von den Sollwerten können frühzeitig erkannt und automatisch oder manuell korrigiert werden, bevor das Reinigungsergebnis verschlechtert wird. Zum anderen werden die Meßdaten vorzugsweise an einen entfernten Ort übertragen, so daß Bedienungs- oder Aufsichtspersonal auch dann laufend über den Zustand des Reinigungsbades informiert ist, wenn es sich nicht in dessen unmittelbarer Nähe befindet. Der Personalaufwand für Kontrolle und Steuerung des Reinigungsbades kann hierdurch beträchtlich reduziert werden. Durch die Dokumentation der im erfindungsgemäßen Verfahren erhobenen Daten wird den Anforderungen einer modernen Qualitätssicherung Rechnung getragen. Der Chemikalienverbrauch kann dokumentiert und optimiert werden.

## Patentansprüche

1. Verfahren zur kontrolle von Reinigerbädern mittels automatischer Bestimmung des Gehalts der wäßrigen Reinigungslösung an anorganisch und/oder organisch gebundenem Kohlenstoff, wobei man programmgesteuert
a) aus der wäßrigen Reinigungslösung eine Probe mit einem vorgegebenen Volumen zieht,
b) erwünschtenfalls die Probe von Feststoffen befreit und/oder homogenisiert,
c) erwünschtenfalls die Probe in einem vorgegebenen oder als Ergebnis einer Vorbestimmung ermittelten Verhältnis mit Wasser verdünnt,
d) den anorganisch und/oder organisch gebundenen Kohlenstoff mit an sich bekannten Verfahren bestimmt,
e) das Ergebnis der Bestimmung an einen entfernten Ort überträgt und ausgibt und/oder auf einem Datenträger speichert und/oder als Basis für weitere Berechnungen heranzieht und
f1) bei Überschreiten eines vorgegebenen Höchstwertes oder bei einer vorgegebenen Änderung des Gehaltes an anorganisch und/oder organisch gebundenem Kohlenstoff oder auf Anforderung eine Einrichtung aktiviert, die eine oder mehrere Ergänzungskomponenten in die Reinigungslösung dosiert, oder
f2) bei Überschreiten eines vorgegebenen Höchstwertes oder bei einer vorgesehenen Änderung des Gehaltes an anorganisch und/oder organisch gebundenem Kohlenstoff Badpflegemaßnahmen einleitet, die den Gehalt der Reinigungslösung an anorganisch und/oder organisch gebundenem Kohlenstoff verringern.

2. Verfahren nach Anspruch 1, **dadurch gekennzeichnet, daß** man im Teilschritt d) anorganisch und/oder organisch gebundenen Kohlenstoff dadurch bestimmt, daß man den Kohlenstoff der Probe in CO₂ überführt und das gebildete CO₂ quantitativ bestimmt.

3. Verfahren nach Anspruch 2, **dadurch gekennzeichnet, daß** man im Teilschritt d) das CO₂ quantitativ bestimmt, indem man es in einer Absorberlösung oder an einem festen Absorber absorbiert und mindestens eine der folgenden Größen mißt Änderung der elektrischen Leitfähigkeit, Restalkalität, Gewichtszunahme.

4. Verfahren nach Anspruch 2, **dadurch gekennzeichnet, daß** man im Teilschritt d) das CO₂ durch Messung der Infrarotabsorption quantitativ bestimmt.

5. Verfahren nach Anspruch 4, **dadurch gekennzeichnet, daß** man die Messung der Infrarotabsorption bei einer Wellenlänge von 4,26 µm entsprechend einer Wellenzahl von 2349 cm⁻¹ durchführt.

6. Verfahren nach Anspruch 4, **dadurch gekennzeichnet, daß** man zur Messung der Infrarotabsorption ein nichtdispersives Photometer einsetzt.

7. Verfahren nach Anspruch 1, **dadurch gekennzeichnet, daß** man im Teilschritt d) den in Form lipophiler Stoffe vorliegenden organisch gebundenen Kohlenstoff bestimmt, indem man die lipophilen Stoffe in ein mit Wasser nicht in jedem Verhältnis mischbares organisches Lösungsmittel extrahiert und sie gravimetrisch nach Abdampfen des Lösungsmittels oder über Infrarotabsorption der lipophilen Stoffe im Extrakt photometrisch bestimmt.

8. Verfahren nach Anspruch 7, **dadurch gekennzeichnet, daß** man die Infrarotabsorption der lipophilen Stoffe im Extrakt bei einer charakteristischen Schwingungsfrequenz der CH₂-Gruppe mißt.

9. Verfahren nach einem oder mehreren der Ansprüche 1 bis 8, **dadurch gekennzeichnet, daß** man die Teilschritte a) bis e) nach einem vorgegebenen Zeitintervall wiederholt.

10. Verfahren nach einem oder mehreren der Ansprüche 1 bis 8, **dadurch gekennzeichnet, daß** man die Teilschritte a) bis e) nach um so kürzeren Zeitintervallen wiederholt, je stärker sich die Ergebnisse zweier aufeinanderfolgender Bestimmungen unterscheiden.

11. Verfahren nach einem oder mehreren der Ansprüche 1 bis 8,**dadurch gekennzeichnet, daß** man die Teilschritte a) bis e) aufgrund einer externen Anforderung ausführt.

12. Verfahren nach einem oder mehreren der Ansprüche 1 bis 11, **dadurch gekennzeichnet, daß** man zur Bestimmung des Gehalts an organisch gebundenem Kohlenstoff vor dem Teilschritt d) anorganisch gebundenen Kohlenstoff aus der Probe entfernt, indem man die Probe ansäuert und das gebildete CO₂ mit einem Gas ausbläst.

13. Verfahren nach einem oder mehreren der Ansprüche 1 bis 12, **dadurch gekennzeichnet, daß** man vor dem Teilschritt d) flüchtige organische Verbindungen durch Ausblasen mit einem Gas aus der Probe entfernt.

14. Verfahren nach einem oder mehreren der Ansprüche 1 bis 13, **dadurch gekennzeichnet, daß** man nach einem vorgegebenen Zeitintervall oder nach einer vorgegebenen Anzahl von Bestimmungen oder aufgrund einer externen Anforderung durch Kontrollmessung einer oder mehrerer Standardlösungen die Funktionsfähigkeit der verwendeten Meßeinrichtung überprüft.

15. Verfahren nach einem oder mehreren der Ansprüche 1 bis 13, **dadurch gekennzeichnet, daß** man durch Kontrollmessung einer oder mehrerer Standardlösungen die Funktionsfähigkeit der verwendeten Meßeinrichtung überprüft, wenn die Ergebnisse zweier aufeinanderfolgender Messungen um einen vorgegebenen Betrag differieren.

16. Verfahren nach einem oder beiden der Ansprüche 14 und 15, **dadurch gekennzeichnet, daß** man je nach Ergebnis der Überprüfung der verwendeten Meßeinrichtung die zwischen der aktuellen und der vorhergehenden Kontrollmessung erfolgten Bestimmungen des Gehaltes an anorganisch und/oder organisch gebundenem Kohlenstoff mit einem Statuskennzeichen versieht, das die Zuverlässigkeit dieser Bestimmungen des Gehaltes an anorganisch und/oder organisch gebundenem Kohlenstoff kennzeichnet.

17. Verfahren nach einem oder beiden der Ansprüche 14 und 15, **dadurch gekennzeichnet, daß** man je nach Ergebnis der Überprüfung der verwendeten Meßeinrichtung mit der automatischen Bestimmung des Gehaltes an anorganisch und/oder organisch gebundenem Kohlenstoff fortfährt und/oder eine oder mehrere der folgenden Aktionen durchführt: Analyse festgestellter Abweichungen, Korrektur der Meßeinrichtung, Beenden der Bestimmungen des Gehaltes an anorganisch und/oder organisch gebundenem Kohlenstoff, Senden einer Statusmeldung oder eines Alarmsignals an ein übergeordnetes Prozeßleitsystem oder an eine Überwachungseinrichtung.

18. Verfahren nach einem oder mehreren der Ansprüche 1 bis 17, **dadurch gekennzeichnet, daß** man die Füllstände und/oder den Verbrauch der verwendeten Lösungen automatisch überwacht und bei Unterschreiten eines vorgegebenen Mindestfüllstandes eine Warnmeldung ausgibt

19. Verfahren nach einem oder mehreren der Ansprüche 1 bis 18, **dadurch gekennzeichnet, daß** man die Ergebnisse der Bestimmungen und/oder der Kontrollmessungen und/oder der Kalibrierungen und/oder die Statussignale kontinuierlich oder in vorgegebenen Zeitabständen und/oder auf Anforderung an einen von dem Ort der Bestimmung unterschiedlichen Ort überträgt.

## Claims

1. A process for monitoring cleaning baths by automatic determination of the content of inorganically and/or organically bound carbon in the water-containing cleaning solution, **characterized in that**, under program control,
a) a sample of predetermined volume is taken from the water-containing cleaning solution,
b) if desired, the sample is freed from solids and/or homogenized,
c) if desired, the sample is diluted with water in a predetermined ratio or in a ratio determined as the result of a preliminary determination,
d) the inorganically and/or organically bound carbon is determined by methods known per se,
e) the result of the determination is transmitted to a remote location and put out and/or is stored on a data carrier and/or used as the basis for further calculations and
f1) if a predetermined maximum value is exceeded or in the event of a predetermined change in the content of inorganically and/or organically bound carbon or on demand, a device is activated to add one or more regenerating components to the cleaning solution or
f2) if a predetermined maximum value is exceeded or in the event of a predetermined change in the content of inorganically and/or organically bound carbon, bath care measures are initiated to reduce the content of inorganically and/or organically bound carbon in the cleaning solution.

2. A process as claimed in claim 1, **characterized in that**, in step d), inorganically and/or organically bound carbon is determined by converting the carbon of the sample into CO₂ and quantitatively determining the CO₂ formed.

3. A process as claimed in claim 2, **characterized in that**, in step d), the CO₂ is quantitatively determined by absorption in an absorber solution or onto a solid absorber and measuring at least one of the following variables: change in electrical conductivity, residual alkalinity, increase in weight.

4. A process as claimed in claim 2, **characterized in that**, in step d), the CO₂ is quantitatively determined by measuring the infrared absorption.

5. A process as claimed in claim 4, **characterized in that** the infrared absorption is measured at a wavelength of 4.26 µm corresponding to a wave number of 2349 cm⁻¹

6. A process as claimed in claim 4, **characterized in that** a nondispersive photometer is used to measure the infrared absorption.

7. A process as claimed in claim 1, **characterized in that**, in step d), the organically bound carbon present in the form of lipophilic substances is determined by extracting the lipophilic substances into an organic solvent which is not miscible with water in any ratio and determining them either gravimetrically after evaporation of the solvent or photometrically through infrared absorption of the lipophilic substances in the extract.

8. A process as claimed in claim 7, **characterized in that** the infrared absorption of the lipophilic substances in the extract is measured at a characteristic vibration frequency of the CH₂ group.

9. A process as claimed in one or more of claims 1 to 8, **characterized in that** steps a) to e) are repeated after a predetermined time interval.

10. A process as claimed in one or more of claims 1 to 8, **characterized in that** steps a) to e) are repeated after time intervals that are shorter, the greater the difference between the results of two successive determinations.

11. A process as claimed in one or more of claims 1 to 8, **characterized in that** steps a) to e) are carried out in response to an external demand.

12. A process as claimed in one or more of claims 1 to 11, **characterized in that**, to determine the content of organically bound carbon before step d), inorganically bound carbon is removed from the sample by acidifying the sample and blowing out the CO₂ formed with a gas.

13. A process as claimed in one or more of claims 1 to 12, **characterized in that** volatile organic compounds are removed before step d) by being blown out from the sample with a gas.

14. A process as claimed in one or more of claims 1 to 13, **characterized in that** the functional reliability of the measuring arrangement used is tested after a predetermined time interval or after a predetermined number of determinations or in response to an external demand by control measurement of one or more standard solutions.

15. A process as claimed in one or more of claims 1 to 13, **characterized in that** the functional reliability of the measuring arrangement used is tested by control measurement of one or more standard solutions when the results of two successive measurements differ by a predetermined amount.

16. A process as claimed in one or both of claims 14 and 15, **characterized in that**, depending on the result of the test on the measuring arrangement used, the determinations of the inorganically and/or organically bound carbon content between the current and the preceding control measurement are provided with a status sign which characterizes the reliability of these determinations of the inorganically and/or organically bound carbon content.

17. A process as claimed in one or both of claims 14 and 15, **characterized in that**, depending on the result of the test on the measuring arrangement used, automatic determination of the inorganically and/or organically bound carbon content is continued and/or one or more of the following actions is carried out: analysis of deviations detected, correction of the measuring arrangement, termination of the determinations of the inorganically and/or organically bound carbon content, transmission of a status report or an alarm signal to a superordinate process control system or to a monitoring system.

18. A process as claimed in one or more of claims 1 to 17, **characterized in that** the filling levels and/or the consumption of the solutions used is automatically monitored and, when the filling level falls below a minimum level, a warning signal is released.

19. A process as claimed in one or more of claims 1 to 18, **characterized in that** the results of the determinations and/or the control measurements and/or the calibrations and/or the status signals are transmitted continuously or at predetermined time intervals and/or on demand to a location different from the point of determination.

## Revendications

1. Procédé pour le contrôle de bains de nettoyage au moyen d'une détermination automatique de la teneur en carbone lié inorganiquement et/ou organiquement de la solution nettoyante aqueuse, dans lequel, par commande par un programme,
a) on soutire de la solution nettoyante aqueuse un échantillon présentant un volume prédéterminé,
b) on libère si souhaité l'échantillon des substances solides et/ou on l'homogénéise,
c) on dilue si souhaité l'échantillon avec de l'eau dans un rapport prédéfini ou déterminé en fonction d'un résultat d'une détermination préalable,
d) on détermine le carbone lié inorganiquement et/ou organiquement par des procédés connus en soi,
e) on transfère et on sort le résultat de la détermination en un lieu éloigné et/ou on l'enregistre sur un support de données et/ou on l'exploite comme base pour d'autres calculs et
f1) on active un dispositif, dans le cas d'un dépassement d'une valeur maximale prédéterminée ou dans le cas d'une modification prédéfinie de la teneur en carbone lié inorganiquement et/ou organiquement ou sur demande, qui dose un ou plusieurs composants complémentaires dans la solution nettoyante ou
f2) on démarre des mesures de soin du bain, dans le cas d'un dépassement d'une valeur maximale prédéterminée ou dans le cas d'une modification prédéfinie de la teneur en carbone lié inorganiquement et/ou organiquement, qui diminuent la teneur en carbone lié inorganiquement et/ou organiquement de la solution nettoyante.

2. Procédé selon la revendication 1, **caractérisé en ce qu'**on détermine dans l'étape partielle d) le carbone lié inorganiquement et/ou organiquement **en ce qu'**on transforme le carbone de l'échantillon en CO₂ et qu'on détermine quantitativement le CO₂ formé.

3. Procédé selon la revendication 2, **caractérisé en ce qu'**on détermine quantitativement dans l'étape partielle d) le CO₂ **en ce qu'**on l'absorbe dans une solution absorbante ou sur un absorbant solide et on mesure au moins l'une des valeurs suivantes : modification de la conductibilité électrique, alcalinité résiduelle, augmentation du poids.

4. Procédé selon la revendication 2, **caractérisé en ce qu'**on détermine quantitativement dans l'étape partielle d) le CO₂ par mesure de l'absorption des infrarouges.

5. Procédé selon la revendication 4, **caractérisé en ce qu'**on effectue la mesure de l'absorption des infrarouges à une longueur d'onde de 4,26 µm, correspondant à un nombre d'onde de 2349 cm⁻¹.

6. Procédé selon la revendication 4, **caractérisé en ce qu'**on utilise pour la mesure de l'absorption des infrarouges un photomètre non dispersif.

7. Procédé selon la revendication 1, **caractérisé en ce qu'**on détermine dans l'étape partielle d) le carbone lié organiquement se trouvant sous forme de substances lipophiles, **en ce qu'**on extrait les substances lipophiles dans un solvant organique non miscible en toute proportion avec de l'eau et on le détermine par gravimétrie après évaporation du solvant ou par photométrie via l'absorption des infrarouges des substances lipophiles dans l'extrait.

8. Procédé selon la revendication 7, **caractérisé en ce qu'**on mesure l'absorption des infrarouges des substances lipophiles dans l'extrait à une fréquence d'oscillation caractéristique du groupe CH₂.

9. Procédé selon l'une quelconque ou plusieurs des revendications 1 à 8, **caractérisé en ce qu'**on répète les étapes partielles a) à e) après un intervalle de temps prédéfini.

10. Procédé selon l'une quelconque ou plusieurs des revendications 1 à 8, **caractérisé en ce qu'**on répète les étapes partielles a) à e) après des intervalles de temps d'autant plus courts que les résultats de deux déterminations consécutives diffèrent.

11. Procédé selon l'une quelconque ou plusieurs des revendications 1 à 8, **caractérisé en ce qu'**on effectue les étapes partielles a) à e) sur base d'une demande externe.

12. Procédé selon l'une quelconque ou plusieurs des revendications 1 à 11, **caractérisé en ce qu'**on élimine, pour la détermination de la teneur en carbone lié organiquement, avant l'étape partielle d), le carbone lié inorganiquement de l'échantillon en acidifiant l'échantillon et en évacuant le CO₂ formé en soufflant avec un gaz.

13. Procédé selon l'une quelconque ou plusieurs des revendications 1 à 12, **caractérisé en ce qu'**on évacue de l'échantillon avant l'étape partielle d) les composés organiques volatils en soufflant avec un gaz.

14. Procédé selon l'une quelconque ou plusieurs des revendications 1 à 13, **caractérisé en ce qu'**on teste l'aptitude au fonctionnement du dispositif de mesure utilisé par une mesure de contrôle d'une ou plusieurs solutions standard après un intervalle de temps prédéfini ou après un nombre prédéfini de déterminations ou sur base d'une demande externe.

15. Procédé selon l'une quelconque ou plusieurs des revendications 1 à 13, **caractérisé en ce qu'**on teste l'aptitude au fonctionnement du dispositif de mesure utilisé par une mesure de contrôle d'une ou plusieurs solutions standard lorsque les résultats de deux mesures consécutives diffèrent d'une valeur prédéfinie.

16. Procédé selon l'une quelconque des revendications 14 et 15 ou les deux revendications 14 et 15, **caractérisé en ce qu'**en fonction du résultat du test du dispositif de mesure utilisé, on attribue aux déterminations réalisées entre la mesure de contrôle actuelle et la mesure précédente de la teneur en carbone lié inorganiquement et/ou organiquement, un indice de statut qui **caractérise** la fiabilité de ces déterminations de la teneur en carbone lié inorganiquement et/ou organiquement.

17. Procédé selon l'une quelconque des revendications 14 et 15 ou les deux revendications 14 et 15, **caractérisé en ce qu'**en fonction du résultat du test du dispositif de mesure utilisé, on poursuit avec la détermination automatique de la teneur en carbone lié inorganiquement et/ou organiquement et/ou on effectue une ou plusieurs des actions suivantes: analyse des déviations constatées, correction du dispositif de mesure, fin des déterminations de la teneur en carbone lié inorganiquement et/ou organiquement, envoi d'une signalisation de statut ou d'un signal d'alarme à un système de guidage de procédé supérieur ou à un dispositif de surveillance.

18. Procédé selon l'une ou plusieurs des revendications 1 à 17, **caractérisé en ce qu'**on surveille automatiquement les états de remplissage et/ou la consommation des solutions utilisées et qu'on émet un avertissement lorsqu'on passe au-dessous d'un état de remplissage minimum prédéfini.

19. Procédé selon l'une ou plusieurs des revendications 1 à 18, **caractérisé en ce qu'**on transfère les résultats des déterminations et/ou des mesures de contrôle et/ou des calibrages et/ou les signaux de statut, en continu ou à des intervals dans le temps et/ou sur demande à un lieu différent du lieu de la détermination.
